# EUROPEAN PATENT SPECIFICATION

(11) **EP 2 930 233 B1**
(45) Date of publication and mention of the grant of the patent: **05.02.2020**
(21) Application number: 13860856.7
(22) Date of filing: 06.12.2013
(51) Int. Cl.: C12M 1/00, C12M 1/26, C12M 3/00

(54) **DEVICE FOR AUTOMATICALLY PEELING CELLS AND SYSTEM FOR PEELING CELLS**
VORRICHTUNG ZUM AUTOMATISCHEN ABLÖSEN VON ZELLEN UND SYSTEM ZUM ABLÖSEN VON ZELLEN
DISPOSITIF POUR DÉTACHER AUTOMATIQUEMENT DES CELLULES ET SYSTÈME POUR DÉTACHER LES CELLULES

(30) Priority: 06.12.2012 JP 2012267172
(43) Date of publication of application: 14.10.2015
(73) Proprietor: Kawasaki Jukogyo Kabushiki Kaisha, Kobe-shi, Hyogo 650-8670 (JP)
(72) Inventor: WATAKABE, Keizo, Kobe-shi, Hyogo 650-8670 (JP); SAKURAI, Takashi, Kobe-shi, Hyogo 650-8670 (JP); ITO, Shimpei, Kobe-shi, Hyogo 650-8670 (JP); NAKASHIMA, Katsumi, Kobe-shi, Hyogo 650-8670 (JP)
(74) Representative: Witte, Weller & Partner Patentanwälte mbB
(86) International application number: PCT/JP2013/007203
(87) International publication number: WO 2014/087667

(56) References cited:
- EP-A1- 1 508 612
- EP-A1- 1 661 980
- JP-A- 2006 325 517
- JP-A- 2010 273 603
- US-A1- 2007 292 946
- US-A1- 2011 124 037
- KATSUMI NAKASHIMA ET AL.: 'Automatic Cell Processing for Regenerative Medicine' JOURNAL OF THE SOCIETY FOR BIOSCIENCE AND BIOENGINEERING vol. 85, no. 10, 25 October 2007, JAPAN, pages 432 - 434, XP008160782

## Description

### TECHNICAL FIELD

The present invention relates to an automatic cell detachment device for mechanically detaching cells adhering to the inner bottom surface of a culture vessel from the inner bottom surface and to a cell detachment system using the automatic cell detachment device.

### Background Art

Conventionally, in the process of cultivating cells, those adhering to the inner bottom surface of a culture vessel are detached from the inner bottom surface by using a scraper. In recent years, automatic cell detachment devices capable of automatically performing the detaching work have been developed.

For example, Patent Literature 1 discloses an automatic cell detachment device, which adopts a gate-shaped arm so as to avoid that a drive unit is disposed above a culture vessel placed on a placing board. A scraper is integrally provided on the distal end of the arm. The base of the arm is connected to a lifting/lowering mechanism disposed laterally to the placing board. The lifting/lowering mechanism lifts and lowers the arm. A moving mechanism causes the lifting/lowering mechanism to move horizontally. Further, document US 2011/124037 A1 discloses a method and a device for automated removal of cells. The device comprises a removing tool with a tip for removing the cells at a detected position of the cell colony.

### Citation List

### Patent Literature

PTL 1: Japanese Laid-Open Patent Application Publication No. 2010-273603

### Summary of Invention

### Technical Problem

In the process of cultivating cells, once a scraper is used, it is desired that the scraper be replaced with a new scraper so as to prevent contamination. However, in the case of the automatic cell detachment device disclosed in Patent Literature 1, in order to replace the scraper, it is necessary to manually separate the arm, on which the scraper is integrally provided, from the lifting/lowering mechanism, and replace the arm including the scraper.

In view of the above, an object of the present invention is to provide an automatic cell detachment device capable of automatic replacement of the scraper.

### Solution to Problem

In order to solve the above-described problems, an automatic cell detachment device according to the present invention includes: a placing board, to which a culture vessel is to be fixed; a scraper for scraping away cells adhering to an inner bottom surface of the culture vessel, the scraper being strip-shaped; a chuck including a pair of claws configured to pinch an upper part of the scraper in a thickness direction of the scraper; a lifting/lowering mechanism configured to lift and lower the chuck; a holding stand configured to hold replacement scrapers in a state where an upper part of each replacement scraper is exposed; and a moving mechanism configured to move the chuck between the holding stand and the placing board.

According to the above-described configuration, since the upper part of the scraper is pinched by the claws of the chuck, after the scraper is used, the used scraper can be released by opening the claws. In addition, since the upper part of a replacement scraper is exposed from the holding stand, by moving the chuck to over the holding stand, the chuck can be equipped with the new scraper. Thus, the replacement of the scraper can be performed automatically.

The inner bottom surface of the culture vessel may have a round shape. The scraper have a width less than a radius of the inner bottom surface of the culture vessel. The moving mechanism may be configured to move the chuck on a line that passes through a center of the inner bottom surface of the culture vessel in plan view. The placing board may be configured to rotate about the center of the inner bottom surface of the culture vessel together with the culture vessel. According to this configuration, by rotating the placing board together with the culture vessel by one revolution when the scraper is in contact with the inner bottom surface of the culture vessel, cells adhering to the inner bottom surface can be scraped away along a ring-shaped path. Since the diameter of the ring shape can be changed by moving the chuck, cells can be scraped away from the entire round-shaped inner bottom surface.

The above-described automatic cell detachment device may further include a rotation mechanism configured to rotate the chuck about a vertical center line of the chuck. According to this configuration, even if the rotation of only the culture vessel is not enough to entirely detach the cells, the remaining cells can be detached by utilizing the rotation of the scraper.

Alternatively, the moving mechanism may be configured to move the chuck in two directions perpendicular to each other on a horizontal plane. The automatic cell detachment device may further include a rotation mechanism configured to rotate the chuck about a vertical center line of the chuck. According to this configuration, whatever the shape of the inner bottom surface of the culture vessel, cells can be scraped away from the entire inner bottom surface of the culture vessel.

The above-described automatic cell detachment device may further include a collection box, into which the scraper is fed after the scraper is used, the collection box being disposed between the placing board and the holding stand. According to this configuration, used scrapers can be collectively discarded at once.

A cell detachment system according to the present invention includes: a cover removing device configured to remove a cover covering a culture vessel from the culture vessel; the above-described automatic cell detachment device; a pipette device configured to suck the cells detached from the inner bottom surface of the culture vessel by the automatic cell detachment device together with a culture medium; a cell observing device configured to capture an image of the culture vessel from which the pipette device has sucked the cells and the culture medium, and determines whether or not there are any cells remaining in the culture vessel; and a transfer device configured to transfer the culture vessel among the cover removing device, the automatic cell detachment device, the pipette device, and the cell observing device. The cell detachment system with this configuration makes it possible to realize highly reproducible work without relying on a proficient worker.

### Advantageous Effects of Invention

The present invention makes automatic replacement of the scraper possible.

### Brief Description of Drawings

Fig. 1 is a plan view of a cell detachment system using an automatic cell detachment device according to one embodiment of the present invention.
Fig. 2 is a side view of a cover removing device.
Fig. 3 is a side view of a pipette device.
Fig. 4 is a side view of a cell observing device.
Fig. 5 is a sectional front view of a part of the automatic cell detachment device.
Fig. 6 is a sectional side view of a part of the automatic cell detachment device.
Fig. 7 is a plan view of a placing board.
Fig. 8A and Fig. 8 show a scraper in the process of scraping cells away.

### Description of Embodiments

Hereinafter, embodiments of the present invention are described with reference to the drawings.

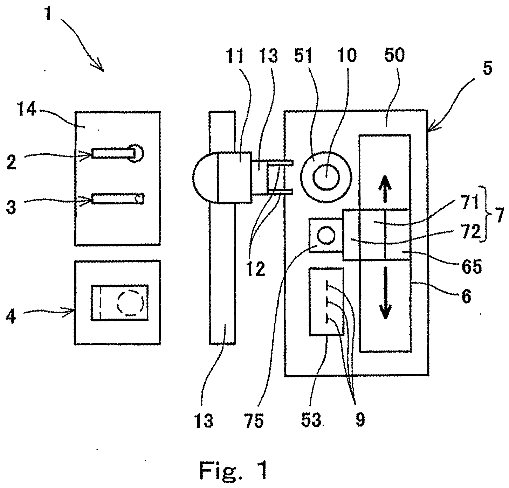

Fig. 1 shows a cell detachment system 1 using an automatic cell detachment device 5 according to one embodiment of the present invention. The cell detachment system 1 includes a cover removing device 2, a pipette device 3, a cell observing device 4, and the automatic cell detachment device 5. Among these devices 2 to 5, a culture vessel 10 is transferred by a robot (corresponding to a transfer device of the present invention) 11.

In the present embodiment, the robot 11 is configured to be runnable along a rail 13. The aforementioned devices 2 to 5 are disposed on both sides of the rail 13. Among these devices, the cover removing device 2 and the pipette device 3 are installed on the same table 14. However, the layout of the devices 2 to 5 is not limited to the one shown in Fig. 1, but may be suitably set. In a case where the devices 2 to 5 are disposed within the work area of the robot 11, the robot 11 may be configured to be non-runnable.

The rail 13 extends to an incubator (not shown) whose internal temperature and humidity are kept constant. The culture vessel 10 is removed from the inside of the incubator by the robot 11. In the present embodiment, the culture vessel 10 is a discoid transparent dish. The robot 11 includes a multi-jointed arm. A hand 13 is mounted to the distal end of the arm. The hand 13 includes a pair of rod-shaped fingers 12, which sandwiches the side of the cell culture vessel 10.

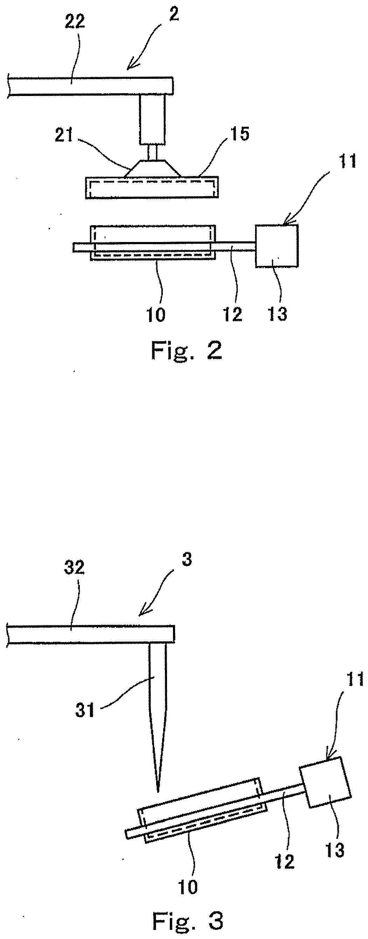

When removed from the inside of the incubator, the culture vessel 10 is in the state of being covered by a cover 15 (see Fig. 2). The cover removing device 2 is a device for removing the cover 15 from the culture vessel 10. More specifically, as shown in Fig. 2, the cover removing device 2 includes a suction pad 21 connected to a vacuum pump, which is not shown. The robot 11 transfers the culture vessel 10 covered by the cover 15 to immediately below the suction pad 21, then lifts the culture vessel 10 such that the cover 15 is sucked and held by the suction pad 21, and thereafter lowers the culture vessel 10. In this manner, the cover 15 is removed from the culture vessel 10. The cover removing device 2 includes a turning mechanism configured to turn an arm 22 supporting the suction pad 21. Each cover 15 sucked and held by the suction pad 21 is stacked up at a storage position adjacent to the suction position. It should be noted that the cover removing device 2 may include a lifting/lowering mechanism for lifting and lowering the suction pad 21.

The culture vessel 10 contains a culture medium, which is a liquid for culturing cells. Cells (adherent cells) adhere to the round inner bottom surface of the culture vessel 10. The automatic cell detachment device 5 is configured to automatically perform cell detaching work of detaching the cells from the inner bottom surface of the culture vessel 10. The configuration of the automatic cell detachment device 5 will be described in detail below.

The pipette device 3 sucks the cells detached from the inner bottom surface of the culture vessel 10 by the automatic cell detachment device 5 together with the culture medium. To be more specific, as shown in Fig. 3, the pipette device 3 includes: a pipette 31 connected to an electric syringe (not shown); and an arm 32 supporting the pipette 31. The robot 11 keeps the culture vessel 10 in an inclined state in a manner to allow the tip of the pipette 31 to be inserted into the culture vessel 10 in the vicinity of the lowest point in the culture vessel 10. Thereafter, the syringe operates, and thereby the culture medium and the cells in the culture vessel 10 are sucked into the pipette 31.

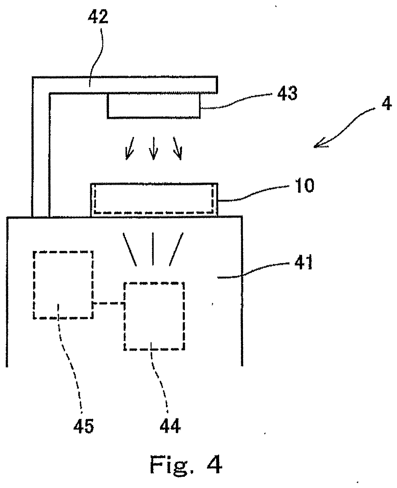

The cell observing device 4 captures an image of the culture vessel 10 from which the pipette device 3 has sucked the culture medium and the cells, and determines whether or not there are any cells remaining in the culture vessel 10. More specifically, as shown in Fig. 4, the cell observing device 4 includes: a body unit 41, on which the culture vessel 10 is to be placed by the robot 11; and an illumination device 43 mounted to the body unit 41 via a bracket 42 and configured to illuminate the culture vessel 10 from above. An image capturing device 44 and an arithmetic operation device 45 are arranged inside the body unit 41. The image capturing device 44 captures an image of the culture vessel 10 illuminated by the illumination device 43. The arithmetic operation device 45 performs image processing of the image captured by the image capturing device 44, and determines whether or not there are any cells remaining in the culture vessel 10. If the arithmetic operation device 45 determines that there are cells remaining in the culture vessel 10, the culture vessel 10 is transferred to the automatic cell detachment device 5 by the robot 11, and the cell detachment is performed again.

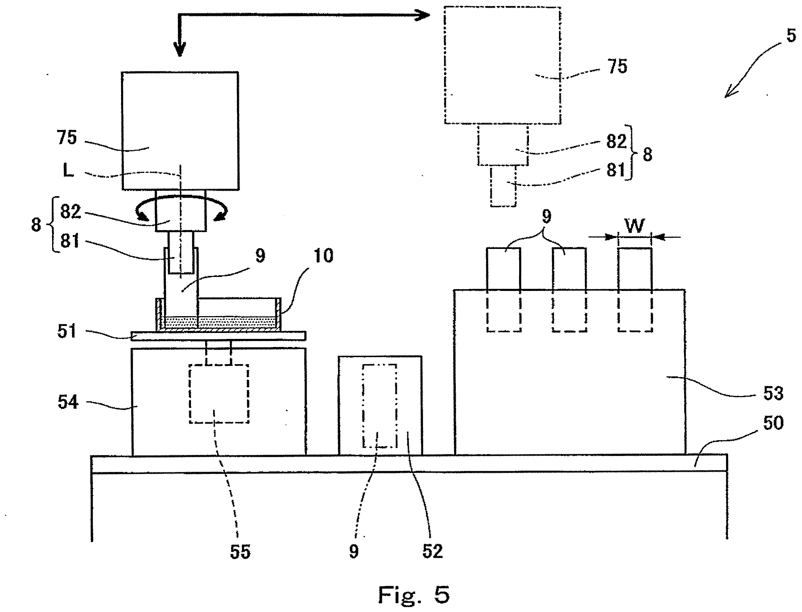

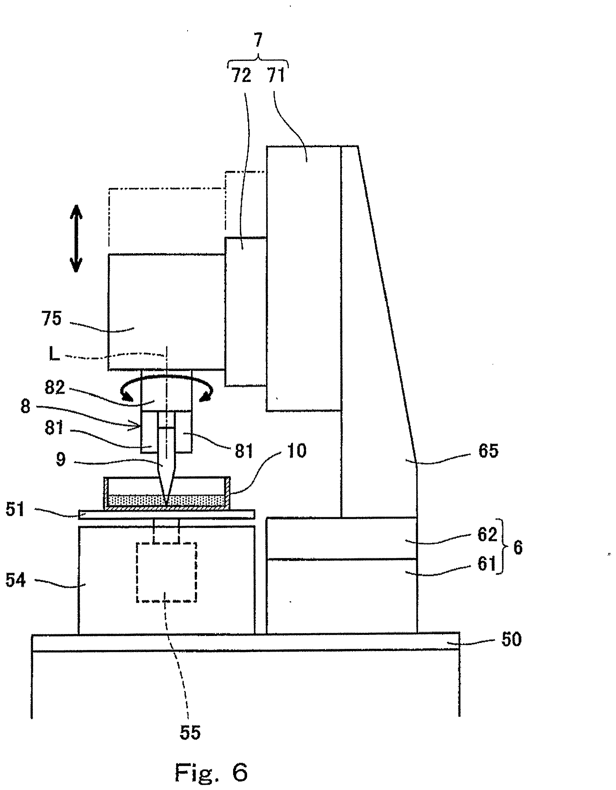

Next, the configuration of the automatic cell detachment device 5 is described in detail with reference to Fig. 5 and Fig. 6.

The automatic cell detachment device 5 includes: a scraper 9 for scraping away cells adhering to the inner bottom surface of the culture vessel 10; and a chuck 8 for holding the scraper 9. The automatic cell detachment device 5 further includes a table 50. A support unit 54, a collection box 52, a holding stand 53, and a moving mechanism 6 are disposed on the table 50.

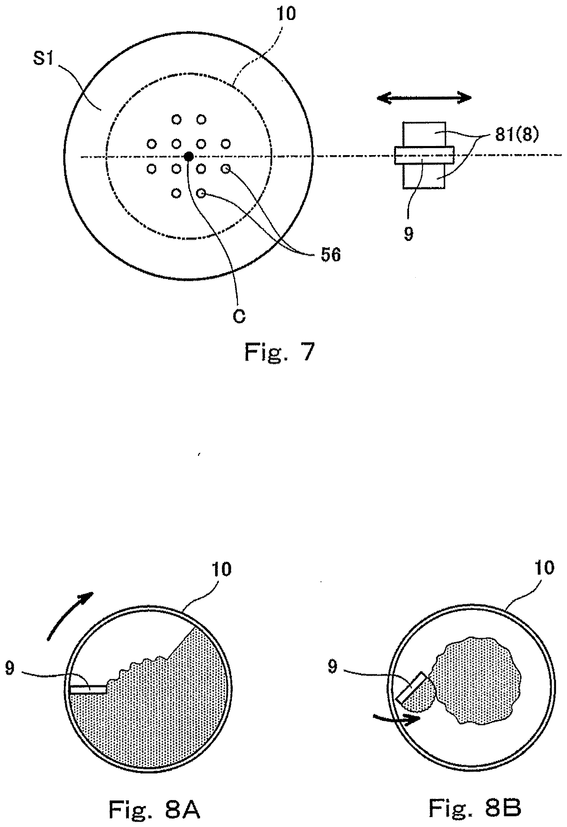

The support unit 54 supports a placing board 51, to which the culture vessel 10 is to be fixed. In the present embodiment, as shown in Fig. 7, the placing board 51 has a discoid shape. The top surface of the placing board 51 is provided with suction holes 56, which are formed in a particular region of the top surface, the region coming into contact with the lower surface of the culture vessel 10. These suction holes 56 are connected to a vacuum pump, which is not shown. Accordingly, the culture vessel 10 is fixed to the placing board 51 by suction. However, the culture vessel 10 may be fixed to the placing board 51 not by suction but by, for example, sandwiching the culture vessel 10 in the horizontal direction.

The robot 11 places the culture vessel 10 on the placing board 51, such that the center C of the inner bottom surface of the culture vessel 10 (see Fig. 7) coincides with the center of the placing board 51. It should be noted that, desirably, the top surface of the placing board 51 is provided with protrusions or recesses for positioning the culture vessel 10.

In the present embodiment, the placing board 51 is configured to rotate together with the culture vessel 10 about the center C of the inner bottom surface of the culture vessel 10. Specifically, the placing board 51 is connected to the output shaft of a motor 55 disposed in the support unit 54. The motor 55 causes the placing board 51 to rotate. It should be noted that the support unit 54 may be formed solely by the motor 55.

The scraper 9 is moved along the inner bottom surface of the culture vessel 10 while being pushed against the inner bottom surface. In the present embodiment, the scraper 9 is strip-shaped and extends vertically. The width W of the scraper 9 is set to be less than the radius of the inner bottom surface of the culture vessel 10. The lower part of the scraper 9 is formed such that the thickness of the scraper 9 gradually decreases downward and the scraper 9 is sharp at the lower end.

The chuck 8 includes: a pair of claws 81 configured to pinch the upper part of the scraper 9 in the thickness direction of the scraper 9; and a body unit 82 configured to operate the claws 81. The chuck 8 is lifted and lowered by a lifting/lowering mechanism 7 between a work position and a retraction position. At the work position, the scraper 9 is pushed against the inner bottom surface of the culture vessel 10. At the retraction position, the scraper 9 is positioned above the culture vessel 10. In the present embodiment, a rotation mechanism 75 is provided between the chuck 8 and the lifting/lowering mechanism 7. The lifting/lowering mechanism 7 lifts and lowers the chuck 8 together with the rotation mechanism 75.

The rotation mechanism 75 rotates the chuck 8 about a vertical center line L of the chuck 8. For example, the rotation mechanism 75 includes a built-in motor whose output shaft is connected to the chuck 8. The lifting/lowering mechanism 7 includes: a drive unit 71 whose axial direction is the vertical direction; and a movable part 72 driven by the drive unit 71. The rotation mechanism 75 is connected to the movable part 72.

The above-described moving mechanism 6 moves the chuck 8 between the placing board 51 and the holding stand 53 together with the rotation mechanism 75 and the lifting/lowering mechanism 7. The moving mechanism 6 includes: a drive unit 61 whose axial direction is the horizontal direction, in which the placing board 51 and the holding stand 53 are spaced apart from each other; and a movable part 62 driven by the drive unit 61. The drive unit 71 of the lifting/lowering mechanism 7 is mounted to the movable part 62 via a bracket 65. In the present embodiment, as shown in Fig. 7, the moving mechanism 6 is configured to move the chuck 8 on a line that passes through the center C of the inner bottom surface of the culture vessel 10.

The holding stand 53 holds replacement scrapers 9 in a state where the upper part of each replacement scraper 9 is exposed. Specifically, the holding stand 53 has a parallelepiped shape, and has a horizontal top surface. Recesses are formed in the top surface of the holding stand 53. Each recess has the same shape as the cross-sectional shape of the scraper 9, and the depth of each recess is less than the height of the scraper 9 (e.g., approximately the half of the height of the scraper 9). The scrapers 9 are fitted in these recesses. In the present embodiment, the width direction of each scraper 9 held by the holding stand 53 is parallel to a direction in which the chuck 8 is moved by the moving mechanism 6. However, as an alternative, the width direction of each scraper 9 held by the holding stand 53 may be diagonal or perpendicular to the moving direction of the chuck 8. It should be noted that supports may be interposed between the holding stand 53 and the table 50, and thereby the holding stand 53 may be positioned away from the table 50 although such a configuration is not shown in the drawings.

For example, the holding stand 53 is disposed at such a height position that the height level of the scrapers 9 held by the holding stand 53 is the same as the height level of the scraper 9 pushed against the inner bottom surface of the culture vessel 10. That is, in this case, when being lowered by the lifting/lowering mechanism 7 to the work position, the chuck 8 can perform both pushing of the scraper 9 to the inner bottom surface of the culture vessel 10 and pinching of a new scraper 9. However, if the lifting/lowering mechanism 7 is capable of stopping the movable part 72 at any intended position, the height position of the holding stand 53 may be any position.

The collection box 52 is disposed between the placing board 51 and the holding stand 53. By opening the claws 81 of the chuck 8 over the collection box 52, the scraper 9 after use is fed into the collection box 52.

Next, one example of operation performed by the automatic cell detachment device 5 is described with reference to the drawings including Figs. 8A and 8B.

In a normal state, the chuck 8 stands by at a retraction position over the holding stand 53. When the culture vessel 10 is placed on the placing board 51 by the robot 11 and fixed to the placing board 51, lowering of the chuck 8 by the lifting/lowering mechanism 7, pinching of a scraper 9 by the claws 81, and lifting of the chuck 8 by the lifting/lowering mechanism 7 are performed. Then, the moving mechanism 6 moves the chuck 8 from over the holding stand 53 to over the placing board 51. Thereafter, the lifting/lowering mechanism 7 lowers the chuck 8. As a result, as shown in Fig. 8A, the scraper 9 is pushed against the inner bottom surface of the culture vessel 10 on the line that passes through the center C of the inner bottom surface. In this state, the placing board 51 is rotated by one revolution. After the placing board 51 is rotated by one revolution, as shown in Fig. 8B, the rotation mechanism 75 rotates the chuck 8 together with the scraper 9, for example, by 90 degrees.

As described above, in the automatic cell detachment device 5 according to the present embodiment, since the upper part of a scraper 9 is pinched by the claws 81 of the chuck 8, after the scraper 9 is used, the used scraper 9 can be released by opening the claws 81. In addition, since the upper part of a replacement scraper 9 is exposed from the holding stand 53, by moving the chuck 8 to over the holding stand 53, the chuck 8 can be equipped with the new scraper 9. Thus, the replacement of the scraper 9 can be performed automatically. Moreover, the cell detachment system 1 using the automatic cell detachment device 5 makes it possible to realize highly reproducible work without relying on a proficient worker.

Further, in the present embodiment, the placing board 51 is configured to rotate. Therefore, by rotating the placing board 51 together with the culture vessel 10 by one revolution when the scraper 9 is in contact with the inner bottom surface of the culture vessel 10, cells adhering to the inner bottom surface can be scraped away from the inner bottom surface along a ring-shaped path. Since the diameter of the ring shape can be changed by moving the chuck 8, cells can be scraped away from the entire round-shaped inner bottom surface.

Stull further, since the automatic cell detachment device 5 according to the present embodiment is provided with the rotation mechanism 75, which rotates the chuck 8, even if the rotation of only the culture vessel 10 is not enough to entirely detach the cells, the remaining cells can be detached by utilizing the rotation of the scraper 9.

Since the collection box 52 is disposed between the placing board 51 and the holding stand 53, used scrapers 9 can be collectively discarded at once.

### (Variations)

The present invention is not limited to the above-described embodiment. Various modifications can be made without departing from the spirit of the invention.

For example, in a case where the moving mechanism 6 is configured to move the chuck 8 in two directions perpendicular to each other on a horizontal plane, the placing board 51 may be configured to be non-rotatable. In this case, whatever the shape of the inner bottom surface of the culture vessel 10, cells can be scraped away from the entire inner bottom surface of the culture vessel 10 by utilizing the two-directional movement by the moving mechanism 6 and the rotation by the rotation mechanism 75.

In the above-described embodiment, the rotation mechanism 75 is provided between the chuck 8 and the lifting/lowering mechanism 7. However, in a case where, for example, a cylinder is used as the lifting/lowering mechanism 7, the rotation mechanism 75 may be mounted to the bracket 65, and the rotation mechanism 75 may cause the chuck 8 to rotate together with the lifting/lowering mechanism 7.

### Reference Signs List

- 1: cell detachment system
- 10: culture vessel
- 11: robot (transfer device)
- 15: cover
- 2: cover removing device
- 3: pipette device
- 4: cell observing device
- 5: automatic cell detachment device
- 51: placing board
- 52: collection box
- 53: holding stand
- 6: moving mechanism
- 7: lifting/lowering mechanism
- 75: rotation mechanism
- 8: chuck
- 81: claw
- 9: scraper

## Claims

1. An automatic cell detachment device (5) comprising:
a placing board (51), to which a culture vessel (10) is to be fixed;
a scraper (9) for scraping away cells adhering to an inner bottom surface of the culture vessel (10), the scraper (9) being strip-shaped;
a chuck (8) including a pair of claws (81) configured to pinch an upper part of the scraper (9) in a thickness direction of the scraper (9);
a lifting/lowering mechanism (7) configured to lift and lower the chuck (8);
a holding stand (53) configured to hold replacement scrapers (9) in a state where an upper part of each replacement scraper (9) is exposed; and
a moving mechanism (6) configured to move the chuck (8) between the holding stand (53) and the placing board (51).

2. The automatic cell detachment device (5) according to claim 1, wherein
the inner bottom surface of the culture vessel (10) has a round shape,
the scraper (9) has a width less than a radius of the inner bottom surface of the culture vessel (10),
the moving mechanism (6) is configured to move the chuck (8) on a line that passes through a center of the inner bottom surface of the culture vessel (10) in plan view, and
the placing board (51) is configured to rotate about the center of the inner bottom surface of the culture vessel (10) together with the culture vessel (10).

3. The automatic cell detachment device (5) according to claim 2, further comprising a rotation mechanism (75) configured to rotate the chuck (8) about a vertical center line of the chuck (8).

4. The automatic cell detachment device (5) according to claim 1, wherein the moving mechanism (6) is configured to move the chuck (8) in two directions perpendicular to each other on a horizontal plane,
the automatic cell detachment device (5) further comprising a rotation mechanism (75) configured to rotate the chuck (8) about a vertical center line of the chuck (8).

5. The automatic cell detachment device (5) according to any one of claims 1 to 4, further comprising a collection box (52), into which the scraper (9) is fed after the scraper (9) is used, the collection box (52) being disposed between the placing board (51) and the holding stand (53).

6. A cell detachment system (1) comprising:
a cover removing device (2) configured to remove a cover (15) covering a culture vessel (10) from the culture vessel (10);
the automatic cell detachment device (5) according to any one of claims 1 to 5;
a pipette device (3) configured to suck the cells detached from the inner bottom surface of the culture vessel (10) by the automatic cell detachment device (5) together with a culture medium;
a cell observing device (4) configured to capture an image of the culture vessel (10) from which the pipette device (3) has sucked the cells and the culture medium, and determines whether or not there are any cells remaining in the culture vessel (10); and
a transfer device (11) configured to transfer the culture vessel (10) among the cover removing device (2), the automatic cell detachment device (5), the pipette device (3), and the cell observing device (4).

## Patentansprüche

1. Automatische Zelltrennvorrichtung (5), welche umfasst:
eine Platzierungsplatte (51), an der ein Kulturgefäß (10) zu befestigen ist;
einen Schaber (9) zum Abschaben von Zellen, die an einer inneren Bodenfläche des Kulturgefäßes (10) haften, wobei der Schaber (9) streifenförmig ist;
ein Spannfutter (8) mit einem Paar Krallen (81), die ausgestaltet sind, einen oberen Teil des Schabers (9) in einer Querrichtung des Schabers (9) einzuklemmen;
einen Hub-/Senkmechanismus (7), der ausgestaltet ist, das Spannfutter (8) anzuheben und abzusenken;
einen Halterungsständer (53), der ausgestaltet ist, Ersatzschaber (9) in einem Zustand zu halten, in dem ein oberer Teil jedes Ersatzschabers (9) freigelegt ist; und
einen Bewegungsmechanismus (6), der ausgestaltet ist, das Spannfutter (8) zwischen dem Halterungsständer (53) und der Platzierungsplatte (51) zu bewegen.

2. Automatische Zelltrennvorrichtung (5) nach Anspruch 1, wobei
die innere Bodenfläche des Kulturgefäßes (10) eine runde Form aufweist,
der Schaber (9) eine Breite aufweist, die kleiner als ein Radius der inneren Bodenfläche des Kulturgefäßes (10) ist,
der Bewegungsmechanismus (6) ausgestaltet ist, das Spannfutter (8) auf einer Linie zu bewegen, die durch eine Mitte der inneren Bodenfläche des Kulturgefäßes (10) in Draufsicht verläuft, und
die Platzierungsplatte (51) ausgestaltet ist, zusammen mit dem Kulturgefäß (10) um die Mitte der inneren Bodenfläche des Kulturgefäßes (10) zu rotieren.

3. Automatische Zelltrennvorrichtung (5) nach Anspruch 2, ferner umfassend einen Rotationsmechanismus (75), der ausgestaltet ist, das Spannfutter (8) um eine vertikale Mittellinie des Spannfutters (8) zu rotieren.

4. Automatische Zelltrennvorrichtung (5) nach Anspruch 1, wobei der Bewegungsmechanismus (6) ausgestaltet ist, das Spannfutter (8) in zwei Richtungen senkrecht zueinander auf einer horizontalen Ebene zu bewegen,
wobei die automatische Zelltrennvorrichtung (5) ferner einen Rotationsmechanismus (75) umfasst, der ausgestaltet ist, das Spannfutter (8) um eine vertikale Mittellinie des Spannfutters (8) zu rotieren.

5. Automatische Zelltrennvorrichtung (5) nach einem der Ansprüche 1 bis 4, ferner umfassend einen Sammelbehälter (52), in den der Schaber (9) nach Verwendung des Schabers (9) eingeführt wird, wobei der Sammelbehälter (52) zwischen der Platzierungsplatte (51) und dem Halterungsständer (53) angeordnet ist.

6. Zelltrennsystem (1), welches umfasst:
eine Abdeckungsentfernungsvorrichtung (2), die ausgestaltet ist, eine Abdeckung (15), die ein Kulturgefäß (10) abdeckt, von dem Kulturgefäß (10) zu entfernen;
die automatische Zelltrennvorrichtung (5) nach einem der Ansprüche 1 bis 5;
eine Pipettiervorrichtung (3), die ausgestaltet ist, die Zellen anzusaugen, die von der inneren Bodenfläche des Kulturgefäßes (10) durch die automatische Zelltrennvorrichtung (5) zusammen mit einem Kulturmedium abgetrennt wurden;
eine Zellbeobachtungsvorrichtung (4), die ausgestaltet ist, ein Bild des Kulturgefäßes (10) aufzunehmen, in dem die Pipettiervorrichtung (3) die Zellen und das Kulturmedium angesaugt hat, und zu bestimmen, ob noch Zellen in dem Kulturgefäß (10) vorhanden sind oder nicht; und
eine Übertragungsvorrichtung (11), die ausgestaltet ist, das Kulturgefäß (10) zwischen der Abdeckungsentfernungsvorrichtung (2), der automatischen Zelltrennvorrichtung (5), der Pipettiervorrichtung (3) und der Zellbeobachtungsvorrichtung (4) zu übertragen.

## Revendications

1. Dispositif de détachement automatique de cellules (5) comprenant:
une table d'installation (51) sur laquelle doit être fixé un récipient de culture (10);
un racleur (9) pour racler des cellules adhérant à une surface inférieure intérieure du récipient de culture (10), le racleur (9) étant en forme de bande;
un mandrin (8) pourvu d'une paire de griffes (81) configurées pour pincer une partie supérieure du racleur (9) dans une direction d'épaisseur du racleur (9);
un mécanisme de soulèvement/abaissement (7) configuré pour soulever et abaisser le mandrin (8);
un support de maintien (53) configuré pour maintenir les racleurs de remplacement (9) dans un état où une partie supérieure de chaque racleur de remplacement (9) est exposée; et
un mécanisme de déplacement (6) configuré pour déplacer le mandrin (8) entre le support de maintien (53) et la table d'installation (51).

2. Dispositif de détachement automatique de cellules (5) selon la revendication 1, dans lequel,
la surface inférieure intérieure du récipient de culture (10) a une forme ronde,
le racleur (9) a une largeur inférieure à un rayon de la surface inférieure intérieure du récipient de culture (10),
le mécanisme de déplacement (6) est configuré pour déplacer le mandrin (8) sur une ligne qui passe par un centre de la surface inférieure intérieure du récipient de culture (10) en vue en plan, et
la table d'installation (51) est configurée pour tourner autour du centre de la surface inférieure intérieure du récipient de culture (10) conjointement avec le récipient de culture (10).

3. Dispositif de détachement automatique de cellules (5) selon la revendication 2, comprenant en outre un mécanisme de rotation (75) configuré pour faire tourner le mandrin (8) autour d'une ligne centrale verticale du mandrin (8).

4. Dispositif de détachement automatique de cellules (5) selon la revendication 1, dans lequel le mécanisme de déplacement (6) est configuré pour déplacer le mandrin (8) dans deux directions perpendiculaires entre elles sur un plan horizontal,
le dispositif de détachement automatique de cellules (5) comprenant en outre un mécanisme de rotation (75) configuré pour faire tourner le mandrin (8) autour d'une ligne centrale verticale du mandrin (8).

5. Dispositif de détachement automatique de cellules (5) selon l'une quelconque des revendications 1 à 4, comprenant en outre une boîte de collecte (52), dans laquelle le racleur (9) est introduit après l'utilisation du racleur (9), la boîte de collecte (52) étant disposée entre la table d'installation (51) et le support de maintien (53).

6. Système de détachement de cellules (1) comprenant:
un dispositif de retrait de couvercle (2) configuré pour retirer un couvercle (15) recouvrant un récipient de culture (10) du récipient de culture (10);
le dispositif de détachement automatique de cellules (5) selon l'une quelconque des revendications 1 à 5;
un dispositif de pipette (3) configuré pour aspirer les cellules détachées de la surface inférieure intérieure du récipient de culture (10) par le dispositif de détachement automatique de cellules (5) conjointement avec un milieu de culture;
un dispositif d'observation de cellules (4) configuré pour capturer une image du récipient de culture (10) à partir duquel le dispositif de pipette (3) a aspiré les cellules et le milieu de culture, et détermine s'il reste des cellules dans le récipient de culture (10); et
un dispositif de transfert (11) configuré pour transférer le récipient de culture (10) entre le dispositif de retrait de couvercle (2), le dispositif de détachement automatique de cellules (5), le dispositif de pipette (3), et le dispositif d'observation de cellules (4).
